# FASCICULE DE BREVET EUROPEEN

(11) **EP 2 855 353 B1**
(45) Date de publication et mention de la délivrance du brevet: **08.06.2016**
(21) Numéro de dépôt: 13725172.4
(22) Date de dépôt: 27.05.2013
(51) Int. Cl.: C01B 35/10, C07C 31/20, C07C 35/16, C07C 215/10, C07C 55/08

(54) **PREVENTION OU SUPPRESSION DE LA CRISTALLISATION DE L'ACIDE BORIQUE PRESENT DANS UNE PHASE AQUEUSE**
VORBEUGUNG ODER UNTERDRÜCKUNG DER KRISTALLISATION VON BORSÄURE IN EINER WÄSSRIGEN PHASE
PREVENTION OR SUPPRESSION OF CRYSTALLISATION OF BORIC ACID PRESENT IN AN AQUEOUS PHASE

(30) Priorité: 29.05.2012 FR 1254930
(43) Date de publication de la demande: 08.04.2015
(73) Titulaire: Commissariat à l'Énergie Atomique et aux Énergies Alternatives, 75015 Paris (FR)
(72) Inventeur: LONGUET, Laurence, F-37250 Veigne (FR); AUTISSIER, Laurence, F-37550 Saint Avertin (FR); BRIERE, Alix, F-37000 Tours (FR)
(74) Mandataire: Brevalex
(86) Numéro de dépôt international: PCT/EP2013/060879
(87) Numéro de publication internationale: WO 2013/178589

(56) Documents cités:
- WO-A2-2005/094545
- GB-A- 2 446 038
- US-A- 5 928 672
- TAYLOR ET AL: "Triol borates and aminoalcohol derivatives of boric acid", POLYHEDRON, PERGAMON PRESS, OXFORD, GB, vol. 15, no. 19, 1 juin 1996 (1996-06-01), pages 3261-3270, XP022289430, ISSN: 0277-5387, DOI: 10.1016/0277-5387(96)00047-2

## Description

### DOMAINE TECHNIQUE

L'invention se rapporte à l'utilisation d'au moins un composé particulier pour prévenir la cristallisation de l'acide borique présent dans une phase aqueuse, ou supprimer cette cristallisation lorsque celle-ci est déjà initiée.

L'invention trouve application, d'une manière générale, dans tous les domaines dans lesquels l'acide borique est susceptible d'être utilisé et, en particulier, dans les domaines dans lesquels il est nécessaire de disposer rapidement de phases aqueuses contenant de l'acide borique qui ne comportent pas de cristaux d'acide borique, par exemple, dans le domaine de l'industrie nucléaire.

### EXPOSÉ DU PROBLÈME TECHNIQUE

L'acide borique, encore appelé borate d'hydrogène, acide boracique ou acide orthoborique, est un composé chimique de formule brute H₃BO₃ qui est classé parmi les acides de Lewis faibles.

Il est communément utilisé dans les industries sidérurgique et métallurgique, en tant que composant de flux de soudage ou de brasage ; dans les industries textile et des polymères, en tant qu'agent ignifugeant de fibres cellulosiques et de matières plastiques ; dans l'industrie du bois, pour ses propriétés antifongiques et insecticides ; ou encore dans l'industrie nucléaire, par exemple, en tant qu'agent absorbeur de neutrons.

Il est encore utilisé dans des produits destinés au public pour ses propriétés biocides, notamment dans des produits d'hygiène corporelle, cosmétiques ou encore antiseptiques.

L'acide borique se présente sous la forme de cristaux incolores ou d'une poudre blanche qui sont relativement peu hydrosolubles, la solubilité massique de l'acide borique dans l'eau valant théoriquement 47,2 grammes par litre (g/L) à une température de 20 degrés Celsius (°C) et à la pression ambiante.

Cette faible solubilité de l'acide borique, bien que variant en fonction de paramètres tels que le pH et la température de la phase aqueuse ainsi constituée, représente un inconvénient majeur lorsqu'une quantité importante d'acide borique dissoute en phase aqueuse doit être rapidement disponible.

De plus, dans le cas de phases aqueuses aux concentrations très élevées en acide borique, exemple étant celui de solutions aqueuses saturées et sursaturées en acide borique, on peut encore observer l'établissement d'un gradient de concentration et de pH au sein de ces phases, celui-ci favorisant davantage encore le phénomène de cristallisation, qui est alors observé au bout de quelques jours seulement.

Les inventeurs se sont donc fixé pour but de trouver une solution au problème posé par la cristallisation de l'acide borique présent dans une phase aqueuse, celle-ci pouvant déjà être initiée.

### EXPOSÉ DE L'INVENTION

Ce but est atteint par l'invention qui propose, en premier lieu, l'utilisation d'au moins un composé comprenant au moins deux fonctions hydroxyle, le composé étant choisi parmi les alcools, les aminoalcools, les acides carboxyliques et les hydroxyacides, pour prévenir la cristallisation de l'acide borique présent dans une phase aqueuse, ou supprimer cette cristallisation lorsque celle-ci est déjà initiée.

On précise que l'on désigne, par l'expression *"phase aqueuse",* une phase dont le solvant est exclusivement ou quasi exclusivement constitué d'eau, c'est-à-dire une phase dans laquelle la proportion volumique d'eau au sein du solvant de la phase aqueuse est au moins égale à 90 pour cent (%), mieux encore, égale à 95% et, de préférence, égale à 100%, et dans laquelle l'acide borique présent dans la phase est susceptible de cristalliser, ou dans laquelle le phénomène de cristallisation est déjà initié.

Ainsi, conformément à l'invention, l'utilisation d'un tel composé permet-elle d'empêcher, à titre préventif, la cristallisation de l'acide borique présent dans une phase aqueuse.

Dans une optique complémentaire, l'utilisation d'un tel composé permet aussi de supprimer ou, à tout le moins, de réduire fortement, à titre curatif, la présence de cristaux d'acide borique déjà formés dans une phase aqueuse contenant de l'acide borique, en favorisant la solubilisation de ceux-ci.

Étant donné que le composé comprenant au moins deux fonctions hydroxyle est destiné à assurer la stabilisation de phases aqueuses contenant de l'acide borique, celui-ci est, de préférence, choisi parmi les composés dans lesquels le nombre total d'atomes de carbone ne dépasse pas huit, de sorte à présenter un caractère hydrophile suffisant pour pouvoir être utilisé en phase aqueuse.

Auquel cas, le composé peut notamment être choisi parmi les alcools polyhydroxylés en C₂ à C₈, que l'on définit comme des composés organiques comportant au moins deux groupements hydroxyle, chacun d'entre eux étant lié à un atome de carbone tétragonal et jusqu'à deux groupements hydroxyle pouvant être liés à un même carbone tétragonal. Ces alcools polyhydroxylés peuvent être aussi bien saturés qu'insaturés, à chaîne linéaire, ramifiée que cyclique. Avantageusement, ces alcools ne comportent pas de groupement(s) fonctionnel(s) autre(s) que les groupements hydroxyles. On peut notamment citer, à titre d'exemples, l'éthylène glycol, le propylène glycol, le 2,2-diméthylpropane-1,3-diol, le pentaérythritol ou encore le myo-inositol.

Le composé peut encore être choisi parmi les aminoalcools polyhydroxylés en C₂ à C₈, que l'on définit comme des alcools polyhydroxylés en C₂ à C₈ comportant au moins une fonction amine -NR¹R², dans laquelle R¹ et R² désignent chacun, indépendamment l'un de l'autre, un atome d'hydrogène ou un groupe alkyle ayant un nombre d'atomes de carbone ajusté de telle sorte que l'aminoalcool polyhydroxylé soit en C₂ à C₈. Ces aminoalcools polyhydroxylés peuvent être aussi bien saturés qu'insaturés, à chaîne linéaire, ramifiée que cyclique, tels que, par exemple, la diéthanolamine, la triéthanolamine, le 2-amino-2-(hydroxyméthyl)-propane-1,3-diol (ou « *Tris* ») ou encore le 2-[*bis*-(2-hydroxyéthyl)-amino]-2-(hydroxyméthyl)-propane-1,3-diol (ou « *Bis-Tris* »)*.*

Le document WO 2005/094545 A2 décrit un procédé d'inhibition de la cristallisation d'acide borique. Ce procédé comprend les étapes suivantes :
- la dissolution d'acide borique dans un mélange formé par de l'eau et un agent vitrifiant pour former un mélange aqueux,
- le séchage de ce mélange aqueux pour former un résidu vitreux contenant de l'acide borique.

L'agent vitrifiant est choisi parmi les aminoalcols et les aminoacides. Parmi les aminoalcools susceptibles d'être utilisés comme agent vitrifiant, le tris(hydroxylméthyl)aminométhane, qui correspond au 2-amino-2-(hydroxyméthyl)-propane-1,3-diol ou Tris, est décrit.

Toutefois, il ressort clairement de l'enseignement du document WO 2005/094545 A2 que le procédé mis en oeuvre permet d'obtenir l'inhibition de la cristallisation d'acide borique dans le résidu vitreux issu de l'étape de séchage. Une inhibition de la cristallisation d'acide borique dans le mélange aqueux issu de l'étape de dissolution n'est par contre ni décrite, ni même suggérée, dans ce document de l'Etat de la Technique.

Le composé peut encore être choisi parmi les acides dicarboxyliques en C₂ à C₈, ces acides pouvant être aussi bien saturés qu'insaturés, à chaîne linéaire, ramifiée que cyclique. Avantageusement, ces acides dicarboxyliques ne comportent pas de groupement(s) fonctionnel(s) autre(s) que les groupements hydroxyles. On peut notamment citer, à titre d'exemples, les acides oxalique, maléique ou encore malonique.

Le composé peut enfin être choisi parmi les hydroxyacides en C₂ à C₈, que l'on définit comme des acides carboxyliques en C₂ à C₈ comportant au moins un groupement hydroxyle différent de celui de la ou des fonction(s) acide carboxylique portée(s) par le composé. Ces hydroxyacides peuvent être aussi bien saturés qu'insaturés, à chaîne linéaire, ramifiée que cyclique, tels que, par exemple, les acides mévalonique ou quinique.

De manière préférentielle, le composé comprenant au moins deux fonctions hydroxyle comporte de deux à six fonctions hydroxyle.

Auquel cas, le composé comprenant au moins deux fonctions hydroxyle est avantageusement choisi parmi l'acide maléique, le 2,2-diméthylpropane-1,3-diol, le 2-amino-2-(hydroxyméthyl)-propane-1,3-diol, le 2-[*bis*-(2-hydroxyéthyl)-amino]-2-(hydroxyméthyl)-propane-1,3-diol, le myo-inositol et le pentaérythritol.

Parmi ceux-ci, le 2-amino-2-(hydroxyméthyl)-propane-1,3-diol (ou « *Tris* »), le 2-[*bis*-(2-hydroxyéthyl)-amino]-2-(hydroxyméthyl)-propane-1,3-diol (ou « *Bis-Tris* ») et le pentaérythritol sont particulièrement préférés.

Conformément à l'invention, l'utilisation d'un composé comportant deux fonctions hydroxyle est tout spécialement préconisée pour le traitement de phases aqueuses saturées ou sursaturées en acide borique, et susceptibles d'être conservées dans des conditions de température non contrôlée, telles que celles régnant dans des sites d'entreposage.

De ce fait, la concentration massique de la phase aqueuse en acide borique est, de préférence, au moins égale à la valeur de la solubilité massique de l'acide borique à la température que présente cette phase aqueuse contenant de l'acide borique.

Dans le cadre de l'invention, on précise que cette concentration massique est déterminée en considérant l'acide borique sous toutes ses formes physiques, aussi bien l'acide borique dissous dans la phase aqueuse, que l'acide borique qui est éventuellement présent sous forme cristallisée au sein de la phase aqueuse.

En outre, le rapport molaire du composé comprenant deux fonctions hydroxyle à l'ensemble de l'acide borique dissous de manière partielle ou totale dans la phase aqueuse contenant de l'acide borique, est préférentiellement compris dans une plage allant de 0,1 pour 1 à 0,7 pour 1.

Par ailleurs, dans le cadre de leurs travaux, les inventeurs ont pu mettre en évidence une influence de la forme sous laquelle le composé comprenant au moins deux fonctions hydroxyle est utilisé.

En effet, ils ont constaté que l'effet de diminution ou de suppression de la cristallisation de l'acide borique présent dans une phase aqueuse est plus prononcé lorsque le composé est ajouté à cette phase aqueuse sous forme solide plutôt qu'en solution liquide, et ce, même en l'absence de toute mesure (agitation, par exemple) visant à favoriser la dissolution du composé dans la phase aqueuse contenant de l'acide borique.

Aussi, le composé comprenant au moins deux fonctions hydroxyle est-il ajouté, de préférence, sous une forme solide à la phase aqueuse contenant de l'acide borique.

Néanmoins, il est également possible d'ajouter le composé comprenant au moins deux fonctions hydroxyle à la phase aqueuse contenant de l'acide borique, sous une forme liquide et, notamment, sous la forme d'une solution qui est obtenue par dissolution préalable du composé dans le même solvant que celui de la phase aqueuse.

L'utilisation d'un composé comportant au moins deux fonctions hydroxyle, telle que venant d'être décrite, est, en particulier, applicable dans le domaine de l'industrie nucléaire, par exemple à des fins de stabilisation de phases aqueuses contenant de l'acide borique destinées à être utilisées comme milieux absorbeurs de neutrons.

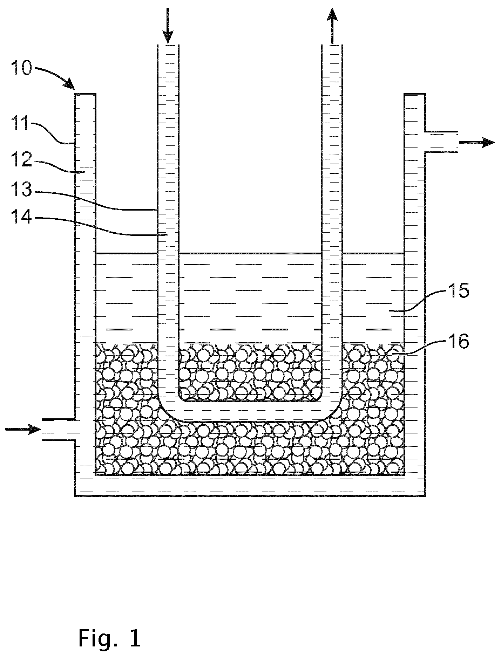

D'autres caractéristiques et avantages de l'invention ressortiront du complément de description qui suit, qui se rapporte à des exemples de composés pouvant être utilisés dans le cadre de l'invention, et à la démonstration des propriétés de prévention ou de suppression de la cristallisation de l'acide borique qui sont permises par ces composés, ce complément de description se référant à la figure annexée.

Il va de soi que ces exemples ne sont donnés qu'à titre illustratif des objets de l'invention et ne constituent en aucun cas une limitation de ces objets.

### BRÈVE DESCRIPTION DES FIGURES

La figure unique est une représentation du dispositif utilisé pour les essais permettant la démonstration des propriétés de prévention ou de suppression de la cristallisation de l'acide borique qui sont permises par l'invention.

### EXPOSÉ DÉTAILLÉ DE MODES DE RÉALISATION PARTICULIERS

### Méthode :

Les essais décrits dans les exemples ci-après sont réalisés à l'aide d'un dispositif du type de celui qui est représenté sur la figure unique.

Le dispositif est constitué d'un bécher 10 à double paroi de verre 11, à l'intérieur de laquelle circule un fluide caloporteur 12. Le bécher 10 contient également un tube métallique 13 en forme de « U », qui contient un fluide caloporteur 14 en circulation.

Les températures des fluides caloporteurs 12 et 14 sont chacune ajustées, l'une de manière indépendante de l'autre, grâce à un générateur de chaleur (non représenté sur la figure unique).

Toutefois, la température du fluide caloporteur 14 est ajustée de manière à ce qu'elle soit toujours strictement inférieure à celle du fluide caloporteur 12, typiquement inférieures de 1°C à 10°C. Si l'on suppose que la conduction thermique du matériau qui compose le tube métallique 13 s'effectue sans perte notable, les parois du tube 13 constituent ainsi un élément dit « *froid ».*

Deux cycles de température peuvent être appliqués à une phase aqueuse 15 contenant de l'acide borique, qui est versée dans le bécher 10.

On définit ainsi un cycle de température « N », par exemple de 28 jours, présentant :
- des périodes de variations thermiques faibles, durant lesquelles les températures des fluides caloporteurs 12 et 14 sont respectivement de (20 ± 2)°C et de (19 ± 2)°C, par exemple valant respectivement 18°C et 17°C de manière simultanée, qui ont pour rôle de faciliter la formation d'un germe d'acide borique par nucléation dans la phase aqueuse étudiée ; et
- des périodes de variations thermiques importantes, durant lesquelles les températures des fluides caloporteurs 12 et 14 sont respectivement de (16 ± 4)°C et de (14 ± 4)°C, par exemple valant respectivement 14°C et 10°C de manière simultanée, qui ont pour rôle d'accélérer la cristallisation de l'acide borique au sein de la phase aqueuse étudiée.

On définit encore un cycle de température « P » (ou « *pénalisant* »), par exemple de 28 jours, présentant :
- des périodes de variations thermiques faibles, durant lesquelles les températures des fluides caloporteurs 12 et 14 sont respectivement de (20 ± 2)°C et de (19 ± 2)°C, par exemple valant respectivement 18°C et 17°C de manière simultanée, qui ont pour rôle de faciliter la formation d'un germe d'acide borique par nucléation dans la phase aqueuse étudiée ;
- des périodes de variations thermiques importantes à des températures supérieures à l'ambiante, durant lesquelles les températures des fluides caloporteurs 12 et 14 sont ainsi respectivement de (35 ± 15)°C et de (30 ± 10)°C, par exemple valant respectivement 50°C et 40°C de manière simultanée ; et
- des périodes de variations thermiques importantes à des températures inférieures à l'ambiante, durant lesquelles les températures des fluides caloporteurs 12 et 14 sont ainsi respectivement de (12 ± 8)°C et de (10 ± 9)°C, par exemple valant respectivement 3°C et 1°C de manière simultanée, qui ont pour rôle d'accélérer la cristallisation de l'acide borique au sein de la phase aqueuse étudiée.

Quel que soit le cycle de température considéré, on définit, à l'issue de l'un de ceux-ci, un taux de cristallisation, qui correspond au rapport de la masse de cristaux d'acide borique relevée à la fin de ce cycle, à la masse totale d'acide borique introduite initialement.

Il est rappelé que, dans les exemples ci-après, la concentration massique d'une solution liquide en une espèce, par exemple dénommée A, est exprimée indépendamment du fait que cette espèce A soit dissoute de manière partielle ou totale dans cette solution.

### Exemple 1 :

Six phases aqueuses notées Pᵢ (l'indice « i » désignant un entier compris entre 1 et 6) sont constituées, chacune par mise en contact :
- d'une phase aqueuse de Tris, de concentration massique Cᵢ et dans laquelle le Tris est sous forme totalement dissoute ; et
- d'une phase aqueuse contenant de l'acide borique, de concentration massique C'ᵢ.

Pour chaque concentration Cᵢ, on calcule la quantité nᵢ de Tris contenue dans un litre de phase aqueuse de Tris.

De même, pour chaque concentration C'ᵢ, on calcule la quantité n'ᵢ d'acide borique contenue dans un litre de phase aqueuse contenant de l'acide borique.

Pour chaque phase Pᵢ, on définit ainsi un rapport molaire Rᵢ (adimensionnel) qui correspond au rapport nᵢ/n'ᵢ. De plus amples informations sont consignées dans le Tableau 1.

**TABLEAU 1**

| Phase aqueuse | Cᵢ (g/L) | C'ᵢ (g/L) | Rᵢ (Ø) |
|---|---|---|---|
| P₁ | 100 | 1490 | 0,034 |
| P₂ | 500 | 1490 | 0,172 |
| P₃ | 50 | 540 | 0,047 |
| P₄ | 100 | 550 | 0,093 |
| P₅ | 200 | 540 | 0,189 |
| P₆ | 500 | 550 | 0,464 |

Chacune des phases aqueuses Pᵢ est soumise à un cycle de température N pendant 28 jours.

À l'issue de cette période, on constate qu'aucune cristallisation n'est survenue au sein de l'une quelconque des phases aqueuses P₁ à P₆.

Les taux de cristallisation sont ainsi égaux à zéro pour l'ensemble des phases P₁ à P₆, bien que l'on puisse formuler les observations suivantes :
- d'une part, dans le cas où la phase P₂ est préparée et homogénéisée avant entreposage, l'acide borique non dissous (pour cause de sursaturation) qui est observé avant réalisation du cycle de température N, est dissous de moitié à l'issue de ce cycle, cela, sans cristallisation supplémentaire, et la phase P₂ présente alors un aspect visuel légèrement trouble ou « cotonneux » ; et
- d'autre part, dans le cas où la phase P₆ est préparée et homogénéisée avant entreposage, l'acide borique non dissous (pour cause de sursaturation) qui est redissous avant réalisation du cycle de température N, ne cristallise pas à l'issue du cycle.

### Exemple 2 :

Une phase aqueuse P₇ contenant de l'acide borique, de concentration massique 550 g/L, est soumise à un premier cycle de température N pendant 28 jours.

À l'issue de cette période, une prise en masse de la phase aqueuse P₇, due à la cristallisation de l'acide borique, est observée. Le taux de cristallisation mesuré pour P₇ est en moyenne de 7%.

Une phase aqueuse P₇, est alors réalisée en introduisant dans la phase aqueuse P₇ et sans agitation, une masse de 80 g de Tris sous forme solide.

En quelques heures, une diminution de la quantité d'acide borique cristallisée est observée.

Un second cycle de température N de 28 jours est alors appliqué à la phase aqueuse P_{7'}, à l'issue duquel aucune cristallisation supplémentaire n'est observée. Le taux de cristallisation pour P_{7'} s'établit alors à 3,1%.

Une diminution appréciable de la cristallisation de l'acide borique dans une phase aqueuse contenant de l'acide borique est ainsi observée en présence de Tris introduit à titre curatif, dans le cas d'un cycle de température N.

### Exemple 3 :

Une phase aqueuse P₈ est constituée par mise en contact d'une phase aqueuse contenant de l'acide borique, de concentration massique 1490 g/L, et d'une phase aqueuse de Tris, de concentration massique 25 g/L et dans laquelle le Tris est sous forme totalement dissoute.

Une phase aqueuse P₉ est constituée par mise en contact d'une phase aqueuse contenant de l'acide borique, de concentration massique 1490 g/L, et de Tris sous forme solide, introduit à hauteur de 25 g par litre de phase aqueuse contenant de l'acide borique.

Chacune des phases aqueuses P₈ et P₉ est soumise à un cycle de température N pendant 28 jours, à l'issue desquels les taux de cristallisation s'établissent à 3,2% pour P₈, et 0,7% pour P₉.

Une diminution appréciable de la cristallisation de l'acide borique dans une phase aqueuse contenant de l'acide borique est ainsi observée lorsque du Tris est utilisé, dans le cas d'un cycle de température N, cette diminution étant davantage marquée lorsque le Tris est introduit sous forme solide.

### Exemple 4 :

Une phase aqueuse P₁₀, contenant de l'acide borique en une concentration massique de 1490 g/L, est constituée.

Une phase aqueuse Pu est constituée par mise en contact d'une phase aqueuse contenant de l'acide borique, de concentration massique 1490 g/L, et de pentaérythritol sous forme solide, introduit à hauteur de 75 g par litre de phase aqueuse contenant de l'acide borique.

Chacune des phases aqueuses P₁₀ et Pu est soumise à un cycle de température N pendant 28 jours, à l'issue desquels cycles les taux de cristallisation s'établissent à 7,2% pour P₁₀, et 2,3% pour P₁₁.

Une diminution appréciable de la cristallisation de l'acide borique dans une phase aqueuse contenant de l'acide borique est ainsi observée en présence de pentaérythritol, dans le cas d'un cycle de température N.

### Exemple 5 :

Une phase aqueuse P₁₂, contenant de l'acide borique en une concentration massique de 1490 g/L, est constituée.

Une phase aqueuse P₁₃ est constituée par mise en contact d'une phase aqueuse contenant de l'acide borique, de concentration massique 1490 g/L, et de pentaérythritol solide, introduit à hauteur de 75 g par litre de phase aqueuse contenant de l'acide borique.

Chacune des phases aqueuses P₁₂ et P₁₃ est soumise à un cycle de température P pendant 28 jours, à l'issue desquels cycles les taux de cristallisation s'établissent à 20,4% pour P₁₂, et 4,0% pour P₁₃.

Une diminution importante de la cristallisation de l'acide borique dans une phase aqueuse contenant de l'acide borique est ainsi observée en présence de pentaérythritol, dans le cas d'un cycle de température P.

## Revendications

1. Utilisation d'au moins un composé comprenant au moins deux fonctions hydroxyle, le composé étant choisi parmi les alcools, les aminoalcools, les acides carboxyliques et les hydroxyacides, pour prévenir la cristallisation de l'acide borique présent dans une phase aqueuse, ou supprimer cette cristallisation lorsque celle-ci est déjà initiée.

2. Utilisation selon la revendication 1, dans laquelle le composé comprenant au moins deux fonctions hydroxyle comporte un nombre total d'atomes de carbone qui est au plus égal à huit.

3. Utilisation selon la revendication 2, dans laquelle le composé comprenant au moins deux fonctions hydroxyle comporte de deux à six fonctions hydroxyle.

4. Utilisation selon la revendication 3, dans laquelle le composé comprenant au moins deux fonctions hydroxyle est choisi parmi l'acide maléique, le 2,2-diméthylpropane-1,3-diol, le 2-amino-2-(hydroxyméthyl)-propane-1,3-diol, le 2-[bis-(2-hydroxyéthyl)-amino]-2-(hydroxyméthyl)-propane-1,3-diol, le myo-inositol et le pentaérythritol.

5. Utilisation selon la revendication 4, dans laquelle le composé comprenant au moins deux fonctions hydroxyle est choisi parmi le 2-amino-2-(hydroxyméthyl)-propane-1,3-diol, le 2-[*bis*-(2-hydroxyéthyl)-amino]-2-(hydroxyméthyl)-propane-1,3-diol et le pentaérythritol.

6. Utilisation selon l'une quelconque des revendications précédentes, dans laquelle la concentration massique de la phase aqueuse en acide borique est au moins égale à la valeur de la solubilité massique de l'acide borique à la température que présente la phase aqueuse contenant de l'acide borique.

7. Utilisation selon l'une quelconque des revendications précédentes, dans laquelle le rapport molaire du composé comprenant au moins deux fonctions hydroxyle à l'acide borique présent dans la phase aqueuse, est compris dans une plage allant de 0,1 pour 1 à 0,7 pour 1.

8. Utilisation selon l'une quelconque des revendications précédentes, dans laquelle le composé comprenant au moins deux fonctions hydroxyle est ajouté sous une forme solide à la phase aqueuse contenant de l'acide borique.

9. Utilisation selon l'une quelconque des revendications 1 à 7, dans laquelle le composé comprenant au moins deux fonctions hydroxyle est ajouté sous une forme liquide à la phase aqueuse contenant de l'acide borique.

## Patentansprüche

1. Verwendung wenigstens einer Verbindung, die wenigstens zwei Hydroxylfunktionen umfasst, wobei die Verbindung ausgewählt ist aus den Alkoholen, den Aminoalkoholen, den Carboxylsäuren und den Hydroxysäuren, zur Verhinderung der Kristallisation von Borsäure, die in einer wässrigen Phase vorhanden ist, oder zur Unterdrückung dieser Kristallisation, wenn sie bereits begonnen hat.

2. Verwendung nach Anspruch 1, bei der die Verbindung, die wenigstens zwei Hydroxylfunktionen umfasst, eine Gesamtzahl von Kohlenstoffatomen umfasst, die höchstens gleich acht ist.

3. Verwendung nach Anspruch 2, bei der die Verbindung, die wenigstens zwei Hydroxylfunktionen umfasst, von zwei bis sechs Hydroxylfunktionen umfasst.

4. Verwendung nach Anspruch 3, bei der die Verbindung, die wenigstens zwei Hydroxylfunktionen umfasst, ausgewählt ist aus Maleinsäure, 2,2-Dimethylpropan-1,3-Diol, 2-Amino-2-(Hydroxymethyl)-Propan-1,3-Diol, 2-[*bis*-(2-Hydroxyethyl)-Amino]-2-(Hydroxymethyl)-Propan-1,3-Diol, Myo-Inositol und Pentaerythritol.

5. Verwendung nach Anspruch 4, bei der die Verbindung, die wenigstens zwei Hydroxylfunktionen umfasst, ausgewählt ist aus 2-Amino-2-(Hydroxymethyl)-Propan-1,3-Diol, 2-[*bis*-(2-Hydroxyethyl)-Amino]-2-(Hydroxymethyl)-Propan-1,3-Diol und Pentaerythritol.

6. Verwendung nach einem der vorhergehenden Ansprüche, bei der die Massenkonzentration der wässrigen Phase an Borsäure wenigstens gleich dem Wert der Massenlöslichkeit der Borsäure bei der Temperatur ist, die die wässrige Phase aufweist, welche die Borsäure enthält.

7. Verwendung nach einem der vorhergehenden Ansprüche, bei der das molare Verhältnis der Verbindung, die wenigstens zwei Hydroxylfunktionen umfasst, zur Borsäure, die in der wässrigen Phase vorhanden ist, in einem Bereich enthalten ist, der von 0,1 zu 1 bis 0,7 zu 1 geht.

8. Verwendung nach einem der vorhergehenden Ansprüche, bei der die Verbindung, die wenigstens zwei Hydroxylfunktionen umfasst, in einer festen Form zu der wässrigen Phase hinzugefügt wird, die die Borsäure enthält.

9. Verwendung nach einem der Ansprüche 1 bis 7, bei der die Verbindung, die wenigstens zwei Hydroxylfunktionen umfasst, in einer flüssigen Form zu der wässrigen Phase hinzugefügt wird, die die Borsäure enthält.

## Claims

1. The use of at least one compound comprising at least two hydroxyl functions, the compound being selected from alcohols, aminoalcohols, carboxylic acids and hydroxyacids, for preventing the crystallization of boric acid present in an aqueous phase, or for suppressing this crystallization when this crystallization has already been initiated.

2. The use according to claim 1, wherein the compound comprising at least two hydroxyl functions includes a total number of carbon atoms which is at most equal to eight.

3. The use according to claim 2, wherein the compound comprising at least two hydroxyl functions includes from 2 to 6 hydroxyl functions.

4. The use according to claim 3, wherein the compound comprising at least two hydroxyl functions is selected from maleic acid, 2,2-dimethylpropane-1,3-diol, 2-amino-2-(hydroxymethyl)-propane-1,3-diol, 2-[*bis*-(2-hydroxyethyl)-amino]-2-(hydroxymethyl)-propane-1,3-diol, myo-inositol and pentaerythritol.

5. The use according to claim 4, wherein the compound comprising at least two hydroxyl functions is selected from 2-amino-2-(hydroxymethyl)-propane-1,3-diol, 2-[*bis*-(2-hydroxyethyl)-amino]-2-(hydroxymethyl)-propane-1,3-diol and pentaerythritol.

6. The use according to any of the preceding claims, wherein the mass concentration of boric acid in the aqueous phase is at least equal to the value of the mass solubility of boric acid at the temperature which is exhibited by this aqueous phase containing boric acid.

7. The use according to any of the preceding claims, wherein the molar ratio of the compound comprising at least two hydroxyl functions to the boric acid present in the aqueous phase is comprised in a range from 0.1/1 to 0.7/1.

8. The use according to any of the preceding claims, wherein the compound comprising at least two hydroxyl functions is added in solid form to the aqueous phase containing boric acid.

9. The use according to any of claims 1 to 7, wherein the compound comprising at least two hydroxyl functions is added in liquid form to the aqueous phase containing boric acid.
